# EUROPEAN PATENT SPECIFICATION

(11) **EP 4 677 618 B1**
(45) Date of publication and mention of the grant of the patent: **08.07.2026**
(21) Application number: 23717106.1
(22) Date of filing: 31.03.2023
(51) Int. Cl.: G16H 40/20, G16H 30/00, G16H 20/40

(54) **GENERATION OF ARTIFICIAL INTRA-OPERATIVE SENSOR DATA**
ERZEUGUNG VON KÜNSTLICHEN INTRAOPERATIVEN SENSORDATEN
GÉNÉRATION DE DONNÉES DE CAPTEUR INTRA-OPÉRATOIRES ARTIFICIELLES

(43) Date of publication of application: 14.01.2026
(73) Proprietor: SNKE OS GmbH, 81829 München (DE)
(72) Inventor: ECKERT, Jacob, 81829 München (DE); SCHMALER, Christian, 81829 München (DE); VILSMEIER, Stefan, 81829 München (DE)
(74) Representative: Maiwald GmbH
(86) International application number: PCT/EP2023/058584
(87) International publication number: WO 2024/199680

(56) References cited:
- WO-A1-2020/172414
- KR-A- 20210 104 190
- US-A1- 2022 076 020
- US-A1- 2022 104 912
- ANONYMOUS: "Data augmentation - Wikipedia", 18 June 2021 (2021-06-18), XP055886526, Retrieved from the Internet <URL:https://en.wikipedia.org/w/index.php?title=Data_augmentation&oldid=1029174861> [retrieved on 20220202]

## Description

### FIELD OF THE INVENTION

The present invention relates to a computer-implemented method of generating artificial intra-operative sensor data usable in training an artificial intelligence (AI) module, training data for training an AI module for automatically analysing real intraoperative sensor data, a computer program, a program storage medium on which said program or said training data is stored, and the present invention relates to an AI module for automatically analysing real intra-operative sensor data.

### TECHNICAL BACKGROUND

Intra-operative data, e.g., surgical data, and especially video data from real surgical procedures is rare. To train AI modules or machine learning models, that shall analyze real intra-operative data, plenty of training data is needed.

In the prior art it was so far suggested to use rendered scenes to learn e.g., instrument segmentation, see e.g., the Hutom Surgical Platform, https://hutom.io/ and https://hsdb-instrument.github.io/. Other technical fields also make use of artificial data for training AI modules. For example, artificial scenes are used in training AI modules for self-driving cars. In detail, in the project of NVIDIA *"NVIDIA DRIVE Sim - Synthetic Data Generation Powered by Omniverse Replicator"* (https://www.youtube.com/watch?v=gPaFgNEF82Q) synthetic 4D ground truth data for training AI modules or deep neural networks that make up the perception systems in autonomous vehicles (AV) is suggested. With DRIVE Sim, synthetic data has proven effective in accelerating AV development, allowing developers to tailor ground truth data to the specific needs of the model.

During their R&D work in the field of digital operating rooms (OR), the inventors of the present invention have identified the need of accurately training an AI module, which after being trained will be configured for automatically identifying or analyzing a real clinical procedure by analysing received real intra-operative sensor data.

Data augmentation, i.e. the generation of additional data for training is generally known, is known from e.g. WO 2020/172414 A1. From WO 2020/172414 A1, data augmentation is known in the context of detection of user presence during surgeries. From WO 2020/172414 A1, data augmentation is known for the recognition of a single medical image.

The present invention has thus the object to provide for an effective method of providing artificial intra-operative sensor data.

Aspects of the present invention, examples and exemplary steps and their embodiments are disclosed in the following. Different exemplary features of the invention can be combined in accordance with the invention wherever technically expedient and feasible.

Technical terms are used by their common sense. If a specific meaning is conveyed to certain terms, definitions of terms will be given in the following in the context of which the terms are used.

### GENERAL DESCRIPTION OF THE INVENTION

The invention is defined by the appended claims.

In this section, a description of the general features of the present invention is given by referring to possible embodiments of the invention

According to a first aspect of the present invention a computer-implemented method of generating artificial intra-operative sensor data usable in training an artificial intelligence (AI) module is presented. The method comprises the following steps:
a) providing a clinical data structure describing several clinical states of a clinical procedure and defining transitions between said several clinical states allowing for different paths of the clinical procedure (step S1);
b) providing at least one geometrical model for modelling procedural scenes in one or more of said clinical states of said clinical procedure (step S2),
   wherein the at least one geometrical model comprises a plurality of geometrical model parameters and boundary conditions for said geometrical model parameters;
   wherein the geometrical model parameters describe said procedural scenes in said clinical states of said clinical procedure,
   wherein a particular combination of values of the geometrical model parameters defines a particular procedural scene of a clinical state of said clinical procedure; the method further comprising the steps
c) defining an individual path of the clinical procedure by selecting at least one transition between a first and a second clinical state of the clinical data structure (step S3);
d) providing, as input for the geometrical model, a set of values of the geometrical model parameters of the at least one geometrical model for said first and said second clinical states of the defined individual path thereby modelling a first individual procedural scene and a second individual procedural scene (step S4), and
e) generating the artificial intra-operative sensor data of said individual path based on the modelled first and second individual procedural scenes (step S5).

Note that the artificial intra-operative sensor data generated in step S5 are not generated by a real measurement using the corresponding real sensor or real sensors. The artificial intra-operative sensor data of the present invention are generated by calculation. Thus, the method step S5 may be understood as calculating said artificial intra-operative sensor data. Thus, as will be appreciated by the skilled reader, the present invention makes use of "artificial intra-operative sensors", since the presented method generates - in a calculative manner - artificial intra-operative sensor data, which would be generated in a real-world situation by sensors installed in an operating room of e.g., a hospital. Thus, with the presented method data from intra-operative sensors is/are simulated. This can be understood as modelling or mimicking data that are generated by said sensors, which are placed within an operating room of e.g., a hospital. It is thus understood by the skilled reader that in the step S5 artificial intraoperative sensor data of artificial intra-operative sensors are generated. This will be detailed in the context of and elucidated with particular embodiments hereinafter.

Preferably the modeled or simulated sensor data have the same or substantially the same quality and properties and/or characteristics as real data, like e.g., noise, bandwith, constraints, and distribution.

As illustrative and non-limiting examples of such intra-operative sensors the following sensors are mentioned: a sound sensor, a photo camera, a video camera, a tracking system for tracking objects within the operating room, particularly an electromagnetic tracking system or an infrared tracking system, a marker-based tracking system, a marker-less tracking system, a temperature sensor, a pressure sensor, a depth camera, a Lidar, a brightness sensor, a microphone, a sound pressure sensor, an x-ray sensor, a magnetic field sensor, an accelerometer, a gyroscope, an ultrasound transducer. In addition, an intra-operative sensor as used herein can be attached or brought in contact with a patient, e.g. to measure vital signs, such as heart rate, one or more pulse parameters, blood pressure, temperature, one or more blood parameters, such blood oxygen level, blood sugar, breathing parameters, one or more electrical properties of tissues (e.g. by implanted or attached electrodes on the brain or skin), neuromonitoring with one or more hand-held sensors, a mechanical motion sensor, a force sensor, a strain sensor, a bladder pressure sensor, a rectal pressure sensor, see also https://en.wikipedia.org/wiki/Monitoring_(medicine)

Furthermore, the step S3 (i.e., defining an individual path of the clinical procedure by selecting at least one transition between a first and a second clinical state of the clinical data structure) emphasizes, particularly when read in combination with the steps S4 and S5, that the method of the present invention generates the desired artificial intraoperative sensor data "along a path" of said clinical procedure defined by said clinical data structure. The presented method thus does not generate single, isolated synthetic scenes of a clinical procedure, but takes into account transitions between two or more clinical states of the clinical data structure, where one scene continuously develops into another scene. In other words, such scenes are connected or interrelated. Thus, in contrast to alternative solutions which only generate individual synthetic scenes of a clinical procedure based on real scenes, but without considering transitions between two clinical states, the generation of the data according to the present invention is carried out along one path within the clinical data structure.

One advantage resulting from this approach is that more complex clinical procedures including or consisting of different connected or interrelated scenes can be modelled or simulated. Thus, when an AI module trained with these generated artificial intraoperative sensor data is used for identifying and/or categorizing a real clinical procedure that is surveyed by sensors, it is possible to use not only the scenes and clinical states, but also the transitions between the involved clinical states for said identification/categorization of the sensed clinical procedure. A method using also the transitions between these clinical states will thus lead to more robust decisions. As is understood by the skilled reader, a specific clinical state or scene can also only be identified or has an importance, if it is put in the context of a procedure and/or a path, i.e., only if step A happened before, step B has a certain meaning. Thus, as will be appreciated by the skilled reader, a state could generally also have a meaning without a context, but for specific states there might be a difference if another state has happened before. In addition, many different variations of clinical procedures, i.e., variations of their development over time, can be considered by varying the path. In this way, more transitions between two clinical states are generated, which cannot be generated based on real data describing single scenes based on a single "base image", for example. With the method of the present invention a model has thus comparatively more information usable for the training of an AI module, since it not only uses the pure, single scenes, but considers one or more transitions. Additionally, it is possible with the presented method to overbalance rare transitions (e.g., special surgical cases), which would also make a solution more robust to edge cases in the real world. In this way, the presented method generates the data along a path and can use all said data to describe an entire clinical procedure, i.e., the entire development over time of said clinical procedure. Therefore, with the presented method rendering the transition/the transition phase between to different clinical states of the clinical data structure is facilitated. Therefore, in a particular embodiment, the method comprises rendering at least one transition between a first and a second clinical state, e.g. one time-dependent "video" is generated.

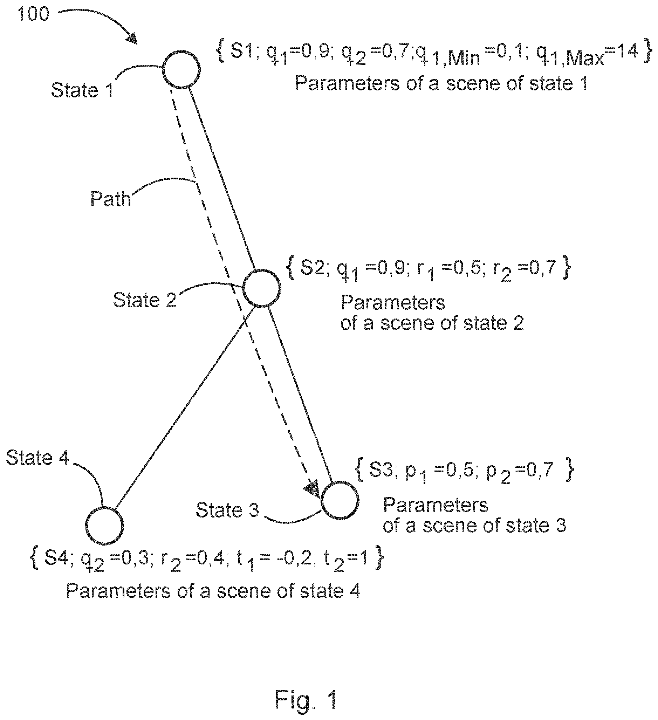

Moreover, the "clinical data structure" as used in the present invention and exemplified for one embodiment in Figure 1, shall be understood as a description of possible courses of a particular clinical procedure along several clinical states or nodes (see states 1 to 4 in Figure, which may be understood as nodes of the structure). The provided clinical data structure thus describes several clinical states of said clinical procedure and defines transitions between said several clinical states, which in turn allows for different paths of the clinical procedure. In one exemplary embodiment, the clinical data structure is embodied as a graph, in which one path comprises a plurality of discrete clinical states or nodes of the clinical procedure. In preferred embodiments, the clinical procedure is a surgical procedure, a diagnostic procedure, and/or a therapeutic procedure, as will be detailed hereinafter in the context of particular embodiments.

The "geometrical model" as used in the present invention shall be understood as a geometrical model that has variable and/or fixed parameters and boundary conditions for these parameters to ensure plausibility and relevance. In one embodiment, the geometric model allows modelling the different courses of action of a particular surgical, diagnostic and/or therapeutic procedure. Varying these parameters of the geometrical model can be used to vary, for example, the scene of the clinical procedure, like for example movement of the objects in said scene, varying the objects present in such a scene, their type, shape, position and/or orientation in the operating room. The geometrical model can be embodied as a function like e.g., y(ax), where the output y is the sensor value, which depends on variable input x.

Regarding the example shown in Figure 1 and described in detail hereinbelow, q1 and q2 shown and used in Figure 1 are examples of a possible parameter, they define the state or properties of the clinical state. x is the variable that would be varied during the simulation of a single clinical state (like e.g., state 1 in Figure 1), e.g., the time, or the position in a cycle, such as a breathing cycle.

It must be noted that at least one geometrical model is provided by the presented method, but also two, three, four, or many more geometrical models may be used for carrying out the method presented herein. For example, in one particular embodiment the same geometrical model is used for each state of the clinical data. Thus, only one geometrical model is used. In another particular embodiment, for each state a different geometrical model is used. Therefore, if the clinical data structure has N number of clinical states, then N number of geometrical models are used in this embodiment. As is understood by the skilled reader, any other possibility using more than one geometrical model and less then N geometrical models is also an embodiment of the present invention. In this case, for some of the clinical states the same geometrical mode is used, whereas for other clinical states a geometrical model is used exclusively.

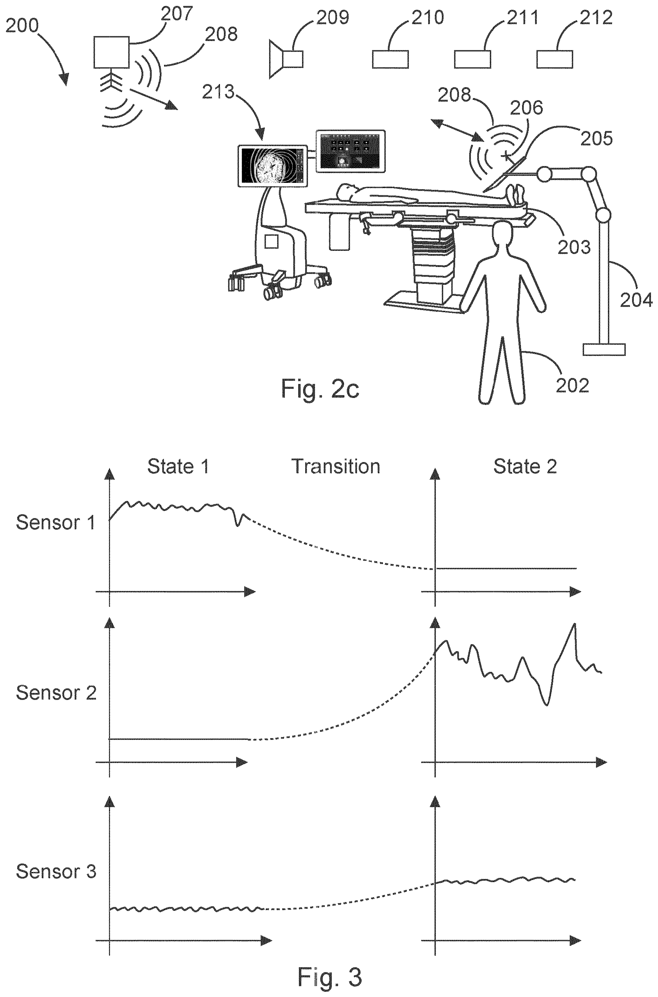

In a preferred embodiment, the geometrical models of the different clinical states depend on each other. For example, many parameters of said two models can be identical to ensure a constant and/or steady transition of one or more sensor values included in the artificial intra-operative sensor data generated with the present invention in step S5. In another preferred embodiment, at least one geometrical model of a first state used in the present invention can have a transition property to a second geometrical model used in the presented method in a second state. In a non-limiting example, ensuring the typical movement of a camera from one position in a first state to another position in a second state can be realized by using a transition property of the geometrical model of the first state. In this way the transition property ensures that the parameter value "camera position" does not jump, i.e., does not have any discontinuity or unsteadiness in the development over time from the first to the second state. As will become apparent from the present disclosure, the present invention may use in this context a spatial registration between a first and second geometrical model. As is understood by the skilled reader, it is desirable that there are smooth transitions of sensor data between different clinical states. This holds true for spatial and non-spatial clinical states. The result of ensuring such smooth transitions is that e.g., a "video" that is rendered along the path is consistent. For example, the color of an object is the same, the position of an object does not "jump", the type of objects remains the same. It should be noted in this context, that there are three parameter types: constant parameters, continuous parameters, and parameters that are free within a path. In the following some examples are provided. A constant parameter may e.g., be the position of the patient over ground in a clinical procedure where it is known beforehand, that in this particular clinical procedure the position of the patient over ground is not changing at all. Further, a continuous parameter may e.g., be that the size of the tumor can only decrease over the course of a tumor resection procedure. Regarding parameters being "free within a path", note that they can be chosen arbitrarily or random at each step, so the model doesn't rely on them, e.g. the position of a door in the wall, or the position of operating room equipment, or the camera position, lighting. This will be explained in more detail in the context of Figure 3.

A "path" of a clinical procedure as used in the present invention shall be understood as a particular, individual sequence of clinical states of/within the clinical data structure. In other words, in the provided clinical data structure, which describes several clinical states of this particular clinical procedure, and which defines transitions between said several clinical states, different paths can be realized, i.e., different possibilities of how this clinical procedure can be realized. A path is thus understood by the skilled reader as one individual course of action or development over time. This will be explained in more detail in the context of the non-limiting embodiment shown in Figure 1, where one possible path is depicted defining a development of the clinical procedure along depicted clinical states 1, 2 and 3, but not along clinical state 4 of the clinical procedure shown in Figure 1. Note, however, that a path may extend in both directions, i.e., back and forth, of a transition between two states. Thus, one possible path in the illustrative example of Figure 1 could be: from clinical state 1 to state 2 to state 3, back to state 2 and then to state 4. Also other variations are possible. As is clear to the skilled reader, a clinical procedure has a typical number of steps or repetitions of steps. In general, the number has to be finite; clinical states can have a maximum number of appearance, a minimum number of appearance, and/or a probability of appearance. Thus, the term "path" as used herein shall be understood as a particular, individual sequence of clinical states of the clinical data structure.

A "clinical procedure" as used in the present invention and as is clear to the skilled reader is a generic description of the kind of medical process or operation. Illustrative examples of different clinical procedures are e.g., the implantation of a hip joint, the implantation of a heart valve, the insertion of DBS (Deep Brain Stimulation) electrodes at a patient's skull, and an appendix operation. It is thus understood as the "genre", "category" or the generic type of surgical, diagnostic and/or therapeutic procedure described by the provided clinical data structure and for which it is desired to get artificial sensor data. Once such artificial sensor data about said clinical procedure are generated with the method presented herein, said data can be used to train an AI module. This subsequently facilitates that said trained AI module can automatically analyse real intra-operative sensor data that are collected during such a real clinical procedure. In this way, the trained AI module can e.g., identify automatically what kind of clinical procedure is going in an operating room that is surveyed by one or more sensors. The real sensor data of such sensors are thus provided to the AI module for said analysis. Thus, the term "clinical procedure" as used herein shall be understood in the context of the present invention as generic description of the kind of medical operation which is simulated with the geometrical model. As mentioned hereinbefore, in preferred embodiments, the clinical procedure is a surgical procedure, a diagnostic procedure, and/or a therapeutic procedure. As will become apparent from the present disclosure, the created artificial intra-operative sensor data at least partly describe the clinical procedure.

The term "clinical state" as used herein shall be understood as a clinically differentiable state, which can thus be differentiated from another clinical state of the associated clinical procedure. Thus, a clinician would consider it a state. Any clinical state used herein may have its own "state label" that can be displayed and/or reported to the user.

The term "procedural scene" or "scene" as used herein shall be understood as a particular intra-operative scene or individual intra-operative situation that can be modelled with a geometrical model suggested herein. A scene "happens" or is associated with one clinical state of the clinical procedure described or defined by the clinical data structure suggested herein. Further, the geometrical models used in the present invention comprise a plurality of geometrical model parameters and boundary conditions for said geometrical model parameters, respectively. Thus, one scene is defined or individualized when each parameter is defined with one value. For example, in the illustrative example of Figure 1, a "procedural scene" or "scene" of clinical state 1 is defined when both parameters q₁ and q₂ are defined or determined. In Figure 1 one particular, individualized scene of clinical state 1 is shown, in which q₁ is set to 0,9 and q₂ is set to be 0,7. q₁ also fulfils the pre-defined boundary conditions that the minimum value for q₁ is set to q₁, _{Min}=0, 1 and that the maximum value for q₁ is set to q₁, _{Max}=14. Thus, with the sensor values q₁=0,9 and q₂=0,7 one procedural scene of clinical state 1 is defined. For example, the parameter q₁ is *"position of the surgeon in the coordinate system of the operating room"* and the parameter q₂ is *"position of the tracked hip-joint implant in the coordinate system of the operating room".* As is appreciate by the skilled reader, the same holds true for procedural scenes defined by the exemplarily chosen parameter values, i.e., the exemplarily chosen sensor values, shown in Figure 1 for clinical states 2, 3 and 4.

Since intra-operative data like surgical data, especially video data, from real surgical procedures is rare, the presented method provides an advantageous method of generating artificial intra-operative sensor data. This generated artificial intra-operative sensor data can be used to train AI modules and machine learning models, that shall analyze real intra-operative data. According to the presented method, this data can be generated with a geometrical model/parametric model that varies the scene, but also the course of the procedure to generate or extract said artificial data. As will become apparent from the present disclosure, the presented method can also use any additional data from other devices, like e.g., data describing the type, shape, reflecting properties of surgical instruments typically used in a particular surgical procedure.

The provided clinical data structure and geometrical model can ensure plausibility and relevance of the clinical procedure scenes described by them. Using the input of e.g., experienced medical personal like surgeons, using medical knowledge from medical databases and/or using suggestions about plausible scenes of a clinical procedure suggested by an AI module is part of particular embodiments in which the clinical data structure and/or the geometrical model are defined or calculated. In other words, the geometrical model may be modelled manually e.g., following clinical guidelines or standard-operating procedures. Alternatively, the geometrical model may be built automatically based on sensor data and/or based on machine learning, e.g., using a hidden Markov-model. Alternatively, the geometric model may be built based on written or structured reports.

As will be explained in more detail hereinafter, the at least one geometrical model of the clinical procedure has parameters and boundary conditions for one or more of these parameters to ensure plausibility and relevance. For creating the desired artificial sensor data, these parameters describing a scene are varied. For example, the movement of the objects in the scene is varied, and/or the type of the objects, their shape, and their position. In an exemplary embodiment, the diameter of a screw used in said clinical procedure varies. Other exemplary parameters of the geometrical modal that can be varied when carrying out the presented method are patient anatomy, the present medical personnel, the background and/or surrounding, the light parameters in the operating room, the sound detectable in the operating room, the temperature in the operating room, and many more.

As is clear to the skilled reader, also different rendering conditions, like for example, artifacts, texture changes, lighting changes, camera position changes, viewing angle and textures of objects being present in the operating room are examples of parameters that can be used by the geometrical model of the present invention.

Therefore, in the presented method artificial intra-operative sensor data relating to the modeled clinical procedure are created based on different clinical states, i.e., set of parameters, of the geometrical model, by varying e.g., the scene including the movement of the objects (e.g., their type, shape, and position) in the scene, by varying the course of the clinical procedure along several surgical states, and by varying the mentioned rendering conditions (e.g., artifacts, texture changes, lighting, camera position, textures of objects, etc.).

The method may include 2D or 3D renderings of plausible videos. In a first category, highly realistic renderings could be used which are not to be distinguished from reality even by experts and which may be involve style transfer. In a second category, high-quality renderings based on a physical, e.g., ray-tracing model of materials and surfaces can be used. In a third category, standard renderings, i.e., geometry-based renderings using textures with heuristic lighting models can be used.

The generation of the artificial intra-operative sensor data may also comprise modelling any other sensor data, e.g., tracking data, or 3D surface point clouds from a physical model or intraoperative x-rays or ultrasound. The generation of the data can be based on a machine-learning model that was trained using real data (for example as DALL-E or DALL-E mini works), possibly using domain randomization to avoid training on the background. This model may also be called a "Generative adversarial network (GAN)" or "generative AI module". Note that also the use of training data from rare cases, also called edge cases, in the context of other situations, that were not part of the training data but modeled, is comprised in an embodiment of the present invention. As is clear to the skilled reader, edge cases are important to use so the product performs well in extreme situations.

As will be described in more detail hereinafter, annotations and/or labels can be generated by the presented method from the model parameters used to generate the artificial data. The annotations and/or labels can be stored together with the generated artificial intra-operative sensor data. The annotations and/or labels used herein may be embodied in examples as a pixelwise layer describing a class, as metadata describing a bounding box, and/or as a label describing the image or the scene or the entire video.

With the so generated artificial intra-operative sensor data, an AI module/ machine-learning algorithm can be trained using said data to later spatially or temporally segment, label or annotate real intra-operative sensor data, i.e., data of the same or similar type.

In the following, exemplary use cases are presented, i.e., possibilities of how to use an AI module that was trained with the data generated by the method of the present invention. Such an AI module is configured for automatically analysing real intra-operative sensor data. Based on the result of this analysis, the following method steps described as uses cases could be carried out. Exemplary use cases of the presented method are the automatic surgical report creation; the calculation of remaining surgery duration; the issuance of warnings; the control of devices or equipment; the controlling other hospital workflows such as OR setup or patient transfer; the re-scheduling of operations; the instrument and disposable checking, tracking, counting; recommending the next surgical/diagnostic/therapeutic step; recommending the next surgical/diagnostic/therapeutic instrument; general real-time, live, or post-op use of the data; and carrying out skill assessment or performance assessment.

The method as presented herein may thus involve the following steps. First, determine or specify the plurality of geometrical model parameters and boundary conditions for said geometrical model parameters. Second, permuting the remaining, undetermined, i.e., free parameters of the geometrical model. This is understood by the skilled reader as a sampling of the parameter space. The result of these permutations/sampling can then be rendered to provide e.g., a video of the simulated/modelled clinical procedure.

It should be noted that some clinical states will look identical in the sensor data, but are different, which can be identified based on the history/state history, i.e., based on the list of states that have occurred before. In other words, this means that a state in a procedure is best described by not only knowing all the sensor data but by knowing states that have occurred before.

According to another exemplary embodiment of the present invention, the method further comprises the steps
storing the generated artificial intra-operative sensor data of said individual path together with the set of values of the geometrical model parameters under which it was generated, wherein the set of values of the geometrical model parameters under which it was generated are stored as training labels; and/or
storing the generated artificial intra-operative sensor data of said individual path together with desired results an AI module trained on the data should produce, wherein the desired results an AI module trained on the data should produce are stored as training labels.

This embodiment requires that the generated artificial intra-operative sensor data are stored with the parameters of the one or more geometrical models under which it was generated or the desired results a model trained on the data should produce, i.e., the training labels. The data and parameters may be stored on the program storage medium of the present invention. As is clear to the skilled reader, this embodiment defines that the generated artificial intra-operative sensor data are generated as data "of said individual path" and thus emphasizes that the presented method creates the data "along the path". As was detailed hereinbefore, this distinguishes the present invention from alternative solutions, which only generate individual synthetic scenes of a clinical procedure based on real scenes, but without considering transitions between two clinical states. The generation of the data according to the present invention is carried out along one path within the clinical data structure.

According to another exemplary embodiment of the present invention, each clinical state of the clinical data structure comprises a label, and wherein each label describes the respective clinical state as a characteristic phase of said clinical procedure and describes it in a clinically differentiable manner.

In this embodiment the labelling of the clinical states described by the clinical data structure is detailed. In order to unambiguously differentiate the clinical states from each other, said labels are characterizing the respective clinical state such that a clinician could clearly consider it a state being different from another state. Therefore, in this embodiment any clinical state has its own "state label" that can be displayed and/or reported to the user. In an embodiment, said labels are displayed and/or reported to the user via a user interface.

Regarding the purpose and advantage of displaying/reporting the label to the user the following should be noted. It has a clinical meaning that the user would derive additional information on or would pay more attention to or compare to. A state could also be defined by set of organs, instruments, or medical devices playing a role in that state, e.g. being moved or being touched or being present. Typically, for a type of procedure, the medical literature will provide a set of states or steps, see e.g., a set of states is defined in the paper that is mentioned by GitHub: https://github.com/CAMMA-public/Surgical-Phase-Recognition

According to another exemplary embodiment the clinical data structure is embodied as a directed and/or weighted graph, preferably with conditions on the directed edges, or (pre-) conditions on the nodes, defining on what must be fulfilled for them to be entered. This embodiment models more explicitly which transitions between states are possible, and preferably have weights defining the likelihood that this transition would happen.

This embodiment also allows defining if backwards transitions (as used in the examples) are possible or not - and therefore if loops are possible. For certain procedures, states can be repeated, for others not, like for example a tumor resection case where it does not make sense or is impossible to go back to "prepare resection" after the "tumor resection" state is finished.

According to another exemplary embodiment of the present invention, for the first clinical state of said individual path a first geometrical model is provided and wherein for the second clinical state of said individual path a second geometrical model is provided, and
wherein the first and second geometrical models are different from another, and/or wherein at least the boundary conditions of the first and second model are different.

In this embodiment it is required that at least two different models are used for two different states. Using more than one geometrical model provides the user with more flexibility in describing with the model the desired clinical procedure in detail. This embodiment makes this clear for two clinical states and two geometrical models. As will be appreciated by the skilled reader, also more, or particularly all geometrical models of all clinical states can be different. In a further embodiment, not only the boundary conditions of the first and second models are different, but also one, more than one, or all geometrical model parameters comprised by said first and second geometrical models can be different.

According to another exemplary embodiment of the present invention, the second geometrical model is spatially registered into the first geometrical model.

In other words, for the case that the first and second geometrical models are adjacent to each other, i.e., "neighbours", within or along a path of the clinical procedure, like for example in Figure 1 states 1 and 2, or states 2 and 3, or states 3 and 4, the spatial registration provides a spatial link between the first and second models. This spatial registration / link between the models ensures smooth transitions of the generated sensor data between adjacent states. Regarding the spatial registration of the two geometrical models, note that the "registration" is limited to a spatial description, e.g., the positions of objects of state 1 and state 2 are continuous or identical. For colors and other non-spatial properties, this is only defined by "indentical" or "continous", not by a registration.

According to another exemplary embodiment of the present invention, the spatial registration of the second geometrical model into the first geometrical model is configured to ensure that first sensor data of at least a first sensor generated for the first clinical state has/have a continuous transition into second sensor data of said at least first sensor generated for the second clinical state.

Based on the previous embodiment, this embodiment makes clear that the desired smooth transition holds true for all adjacent states, in which the respective models use or share at least one sensor of the same kind. **In** general, there should be smooth transitions of sensor data between adjacent or neighboured clinical states for sensors sensing or measuring spatial parameters as well as also for sensors sensing or measuring non-spatial parameters. **In** this way, one can achieve the result that for example a video rendered along the path of the clinical data structure describing the clinical procedure is consistent, e.g., the color of one object is the same; the position of object does not "jump", the type of object remains the same. **In** this context it is again recalled that in principle there are three different parameter types: constant parameters, continuous parameters, and parameters that are free within a path.

According to another exemplary embodiment of the present invention, for all geometrical models of all adjacent clinical states of the clinical data structure, which are used to generate artificial intra-operative sensor data for the same type of artificial intra-operative sensor, said spatial registration of the respective geometrical model is configured to ensure said continuous transition of the generated sensor data of said adjacent clinical state.

**In** other words, this embodiment ensures that for each clinical state, which has an adjacent clinical state, in which one or more of the same type of artificial intra-operative sensor is used, a spatial registration between the respective geometrical models of said two adjacent clinical states is provided. For example, in the non-limiting embodiment shown in Figure 1, in both of the adjacent clinical states 1 and 2 the parameter q₁ is sensed or measured by a corresponding artificial intra-operative sensor. Similarly, in Figure 1, in both of the adjacent clinical states 2 and 4 the parameter r₂ is sensed or measured by a corresponding artificial intra-operative sensor. Therefore, according to the embodiment described hereinbefore, the geometrical model of state 2 in Figure 1 is preferably spatially registered into the geometrical model of state 1 in Figure 1 with respect to parameter q₁. Similarly, in a preferred embodiment the geometrical model of state 4 in Figure 1 is preferably spatially registered into the geometrical model of state 2 with respect to parameter r₂. Hence, data, which are recorded/sensed by the same sensor are having smooth transition between adjacent clinical states.

According to another exemplary embodiment of the present invention, the generated artificial intra-operative sensor data at least comprise tracking data and image data; in particular, tracking data and image data of an intra-operative camera, which is tracked.

Tracking information can provide spatial information about objects placed in the operating room without needing any input from videos or images. With this embodiment, one exactly knows where tools, a patient, and/or one or more cameras etc. are placed, potentially even if they are maybe not visible to a video camera at a specific moment. This embodiment also allows using tracked microscopic videos to know exactly where the focus of this camera was put on, even if one would totally discard the microscopes video itself. This embodiment relates to both the generation of image data and of tracking data. In contrast to alternative solutions using a "base image", the present invention in this embodiment uses tracked positions, preferably tracked central positions, of objects to construct scenes. On huge advantage thereof is, that with the present method one can simulate positions of devices and know also the position in a real world scenario. Thus, one has the exact position of e.g., a microscope in relation to a patient which can also be helpful in identifying a specific procedure step. If one has only a video without any positional data about objects and camera this is comparatively disadvantageous.

According to another exemplary embodiment of the present invention, also artificial intra-operative sensor data of artificial intra-operative sensors are generated, which are not used and/or not involved in said procedural scene of said clinical state of said clinical procedure.

In this embodiment all sensor data are generated, i.e., also for those sensors of any geometrical model of any clinical state of said clinical procedure that are silent, i.e., not involved in that particular clinical state. For example, in the non-limiting example of Figure 1 this would mean that when creating the artificial intra-operative data for the clinical state 1, then also the data of the sensor of parameters r₁ and r₂ (used in the clinical state 2, but not in the clinical state 1), p₁ and p₂ (used in the clinical state 3, but not in the clinical state 1), as well as t₁ and t₂ (used in the clinical state 4, but not in the clinical state 1) would be created with the value 0, since they are not used in clinical state 1. However, that they sensor value is 0 can be of advantage and use when training an AI module. For example, in case the endoscope is not used during the preparation of one particular clinical procedure, this may still be relevant data that could be used training purposes. In other words, also the sensor data is rendered which originates from any "non-used" sensor, i.e., from those sensors that are not involved in the clinical procedure during that clinical state.

According to another exemplary embodiment of the present invention, in step S5 the generation of the artificial intra-operative sensor data is carried out by rendering image data of an intra operative image sensor and/or of an intra-operative video sensor.

In this embodiment, the artificial intra-operative sensor, the data of which are generated in step S5 of the method described herein, is embodied as one of the following: an intra operative image sensor, an intra operative camera, an intra-operative video sensor, and an intra operative video camera. an intra operative

According to another exemplary embodiment of the present invention, the method further comprising the step repeating steps S3 and S4 for several clinical states and for several sets of values of the geometrical model parameters, and repeating step S5 thereby generating a plurality of artificial intra-operative sensor data for a plurality of different paths of the clinical data structure and for a plurality of procedural scenes (step S6).

This embodiment describes that several iterations of steps S3 to S5 can be carried out in order to generate the entire artificial intra-operative sensor data not only along one path, but along several, preferably all possible paths allowed by the clinical data structure. In an example thereof, all possible artificial intra-operative sensor data of all possible procedural scenes of the clinical states 1, 2 and 3 of the path shown in Figure 1 would be created in a first iteration. In a second iteration, e.g., the path starting from clinical state 1 to state 2 to state 3, back to state 2 and then to state 4.

According to another exemplary embodiment of the present invention, artificial intra-operative sensor data are generated for all clinical states of said defined individual path of the clinical procedure.

In an alternative to this embodiment, the presented method may also use inducing real data at one or more clinical states. Thus, a mixture of artificial intra-operative sensor data and real data could be used according to this embodiment.

According to another exemplary embodiment of the present invention, the generated artificial intra-operative sensor data of all clinical states of said defined individual path of the clinical procedure are provided in a chronological sequence such that one or more videos of the clinical procedure is provided.

In this embodiment one or more videos per individual path are generated. Since with the presented methods the data can be generated for several paths, also several videos, one or more per path, can be generated. Each such video can then be used as training data for an AI module, as explained hereinbefore and hereinafter in more detail.

According to another exemplary embodiment of the present invention, all possible procedural scenes in each clinical state are modelled.

According to another exemplary embodiment of the present invention, in step S4 the values of the geometrical parameters are provided by a random generator, or by a mathematical function selecting the values of the geometrical parameter.

This embodiment details how the geometrical parameters could be selected and describes how said parameters can be permuted. The sampling strategy of objects is that all respect the boundary conditions of the geometric model to only generate physically valid configurations. In one example, all possible combinations of valid parameter values are used, and/or a sampling from parameter space (Monte Carlo) is used, and/or a Machine Learning model trained on real observations is used. The sampling strategy could overweight configurations which are underrepresented in real data. This knowledge could be provided by a user and could then be used by the presented method. In a particular embodiment, the method uses probabilities for a transitions between clinical states/nodes. Thus, the selection of the paths can be based on the probability (e.g., directly or using the inverse of the probability).

According to another exemplary embodiment of the present invention, the sensor data are intra-operative video data, and the step S5 of generating the artificial sensor data comprises rendering an artificial video of said clinical procedure.

According to the present invention, the method further comprises the step
g) using the generated artificial intra-operative sensor data and the set of values of the geometric model parameters used during the generation of said artificial intra-operative sensor data, and preferably also the labels derived therefrom and explained herein, for training an AI module for automatically analysing real intra-operative sensor data.

This embodiment specifies the purpose of the artificial data generation, i.e., the training of an AI module. Note that most parameters very most likely will not have an influence on the labels, or even on the generated sensor data (consider e.g., variations of parameters of the geometric model at a location which is occluded to the sensor).

According to the present invention, the method further comprises the steps
h) providing real intra-operative sensor data describing a real clinical procedure to the trained AI module trained with the generated artificial intra-operative sensor data as described herein, and
i) automatically identifying said real clinical procedure by said trained AI module.

According to another exemplary embodiment of the present invention, wherein each transition of the clinical data structure includes a weight associated with a probability or frequency of occurrence in the clinical procedure.

According to another exemplary embodiment of the present invention, the clinical procedure is a surgical procedure, a diagnostic procedure, and/or a therapeutic procedure.

According to another exemplary embodiment of the present invention, for at least two clinical states, preferably for all clinical states, the respective geometrical model, the respective geometrical model parameters and the respective boundary conditions are dependent on the clinical state.

According to another exemplary embodiment of the present invention, the provided geometrical model is configured for spatially modelling procedural scenes in one or more of said clinical states of said clinical procedure. According to another exemplary embodiment of the present invention, wherein the geometrical model parameters define at least a position, a shape and/or a type of one or more objects occurring in said procedural scene of said clinical state of said clinical procedure.

In contrast to other embodiments, in which also parameters about e.g., the operating room illumination and/or colour of an object are used, this embodiment makes use of the spatial parameters position, a shape and/or a type of one or more objects occurring in said procedural scene. Note that the type of an object can be taken from a database of variants, e.g., also containing variants that have not been observed in clinical data yet, e.g., a new device like e.g. a new endoscope that has not been released yet. Thus, the presented method may make use of data available in such medical or surgical databases. It should also be noted that the presented method does not need a base image from real data. Advantageously, the presented method can render scenes / situations, for which no base image, i.e., no real image exists.

According to a second aspect of the present invention a program is presented which, when running on a computer or when loaded onto a computer, causes the computer to perform the method steps of the method according to any of the preceding claims.

The program shall be understood as computer program element. It may be part of a computer program, but it can also be an entire program by itself. For example, the program may be used to update an already existing computer program to get to the present invention. In this second aspect, the invention is directed to a computer program which, when running on at least one processor (for example, a processor) of at least one computer (for example, a computer) or when loaded into at least one memory (for example, a memory) of at least one computer (for example, a computer), causes the at least one computer to perform the above-described method according to the first aspect. The invention may alternatively or additionally relate to a (physical, for example electrical, for example technically generated) signal wave, for example a digital signal wave, carrying information which represents the program, for example the aforementioned program, which for example comprises code means which are adapted to perform any or all of the steps of the method according to the first aspect. A computer program stored on a disc is a data file, and when the file is read out and transmitted it becomes a data stream for example in the form of a (physical, for example electrical, for example technically generated) signal. The signal can be implemented as the signal wave which is described herein. For example, the signal, for example the signal wave is constituted to be transmitted via a computer network, for example LAN, WLAN, WAN, for example the internet. The invention according to the second aspect therefore may alternatively or additionally relate to a data stream representative of the aforementioned program.

Training data for training an AI module for automatically analysing real intra-operative sensor data is presented, wherein the training data were generated according to the method of any of the aspects or embodiments presented herein.

According to a fourth aspect of the present invention a program storage medium is presented, on which said program and/or said training data is stored.

The program storage medium shall be understood as computer readable medium. It may be seen as a storage medium, such as for example, a USB stick, a CD, a DVD, a data storage device, a hard disk, or any other medium on which a program element as described above can be stored. The program of the present invention may be stored/distributed on a suitable medium such as an optical storage medium or a solid-state medium supplied together with or as part of other hardware, but may also be distributed in other forms, such as via the Internet or other wired or wireless telecommunication systems.

In this third aspect, the invention is directed to a non-transitory computer-readable program storage medium on which the program according to the second aspect and/or said training data of the third aspect is/are stored.

According to a fifth aspect of the present invention an AI module for automatically analysing real intra-operative sensor data is presented, wherein the AI module was trained with training data according to the third aspect.

In preferred embodiments, the AI module of the present invention is selected from the group consisting of a generative adversarial network, a convolutional neural network, and a neural network.

According to an exemplary embodiment, the AI module is configured to receive as an input real intra-operative sensor data describing a real clinical procedure, and wherein the AI module is configured for automatically identifying said real clinical procedure by analyzing the received real intra-operative sensor data.

For example, the invention does not involve or in particular comprise or encompass an invasive step which would represent a substantial physical interference with the body requiring professional medical expertise to be carried out and entailing a substantial health risk even when carried out with the required professional care and expertise. For example, the invention does not comprise a step of positioning a medical implant in order to fasten it to an anatomical structure or a step of fastening the medical implant to the anatomical structure or a step of preparing the anatomical structure for having the medical implant fastened to it. More particularly, the invention does not involve or in particular comprise or encompass any surgical, diagnostic or therapeutic activity. For this reason alone, no surgical, diagnostic or therapeutic activity and in particular no surgical, diagnostic or therapeutic step is necessitated or implied by carrying out the invention.

### DEFINITIONS

In this section, definitions for specific terminology used in this disclosure are offered which also form part of the present disclosure.

### Artificial intelligence module

In the context of the present invention, the term "artificial intelligence module" shall comprise machine learning models.

An artificial intelligence (AI) module is an entity that processes one or more inputs into one or more outputs by means of an internal processing chain that typically has a set of free parameters. The internal processing chain may be organized in interconnected layers that are traversed consecutively when proceeding from the input to the output.

Many artificial intelligence modules are organized to process an input having a high dimensionality into an output of a much lower dimensionality. For example, an image in HD resolution of 1920 x 1080 pixels lives in a space having a 1920 x 1080 = 2,073,600 dimensions. A common job for an artificial intelligence module is to classify images into one or more categories based on, for example, whether they contain certain objects. The output may then, for example, give, for each of the to-be-detected objects, a probability that the object is present in the input image. This output lives in a space having as many dimensions as there are to-be-detected objects. Typically, there are on the order of a few hundred or a few thousand to-be-detected objects.

Such a module is termed "intelligent" because it is capable of being "trained." The module may be trained using records of training data. A record of training data comprises training input data and corresponding training output data. The training output data of a record of training data is the result that is expected to be produced by the module when being given the training input data of the same record of training data as input. The deviation between this expected result and the actual result produced by the module is observed and rated by means of a "loss function". This loss function is used as a feedback for adjusting the parameters of the internal processing chain of the module. For example, the parameters may be adjusted with the optimization goal of minimizing the values of the loss function that result when all training input data is fed into the module and the outcome is compared with the corresponding training output data.

The result of this training is that given a relatively small number of records of training data as "ground truth", the module is enabled to perform its job, e.g., the classification of images as to which objects they contain, well for a number of records of input data that higher by many orders of magnitude. For example, a set of about 100,000 training images that has been "labelled" with the ground truth of which objects are present in each image may be sufficient to train the module so that it can then recognize these objects in all possible input images, which may, e.g., be over 530 million images at a resolution of 1920 x 1080 pixels and a color depth of 8 bits.

### Neural network,

A neural network is a prime example of an internal processing chain of an artificial intelligence module. It consists of a plurality of layers, wherein each layer comprises one or more neurons. Neurons between adjacent layers are linked in that the outputs of neurons of a first layer are the inputs of one or more neurons in an adjacent second layer. Each such link is given a "weight" with which the corresponding input goes into an "activation function" that gives the output of the neuron as a function of its inputs. The activation function is typically a nonlinear function of its inputs. For example, the activation function may comprise a "pre-activation function" that is a weighted sum or other linear function of its inputs, and a thresholding function or other nonlinear function that produces the final output of the neuron from the value of the pre-activation function.

### Convolutional neural network,

A convolutional neural network is a neural network that comprises "convolutional layers". In a "convolutional layer", the output of neurons is obtained by applying a convolution kernel to the inputs of these neurons. This greatly reduces the dimensionality of the data. Convolutional neural networks are frequently used in image processing.

### Generative adversarial network,

A generative adversarial network is a combination of two neural networks termed "generator" and "discriminator". Such a network is used to artificially produce records of data that are indistinguishable from records taken from a given set of training records of data. The generator network is trained with the goal of creating, from an input record with random data, an output record that is indistinguishable from the records in the set of training records. I.e., given that output record alone, it cannot be distinguished whether it has been produced by the generator or whether it is contained in the set of training records. The discriminator, in turn, is specifically trained to classify given records of data as to whether they are likely "real" training records or "fake" records produced by the generator. The generator and the discriminator thus compete against each other.

For example, a generative adversarial network may be used to create photorealistic images that are indistinguishable from a set of training images. From a limited number of training images obtained, e.g., by medical imaging, a near-infinite number of fake images that can pass for such medical images can be generated. A prime application of this is the production of training data for other artificial intelligence modules, e.g., modules that are to be trained to classify whether certain features or objects are present in medical images.

### Computer implemented method

The method in accordance with the invention is for example a computer implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services^{™}. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz^{®}, a product of Brainlab AG. The monitor may also be the monitor of a portable, for example handheld, device such as a smart phone or personal digital assistant or digital media player.

The invention also relates to a program which, when running on a computer, causes the computer to perform one or more or all of the method steps described herein and/or to a program storage medium on which the program is stored (in particular in a non-transitory form) and/or to a computer comprising said program storage medium and/or to a (physical, for example electrical, for example technically generated) signal wave, for example a digital signal wave, carrying information which represents the program, for example the aforementioned program, which for example comprises code means which are adapted to perform any or all of the method steps described herein.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer; a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: illustrates an embodiment of a clinical data structure describing several clinical states of a clinical procedure and defining transitions between said several clinical states allowing for different paths of the clinical procedure;
- Figs. 2a to 2c: exemplarily show three different scenes of a surgical procedure in an operating room in form of three different images, which were generated with a method embodiment of the present invention; and
- Fig. 3: is a schematic illustration of different transitions of three sensor values transiting from a first to a second clinical state used in the context of the present invention.

### DESCRIPTION OF EMBODIMENTS

Figure 1 shows an embodiment of a clinical data structure 100, which contains several clinical states (states 1 to 4) of a particular clinical procedure, like e.g., a particular section of the implantation of a hip-joint. This was explained hereinbefore in the context of the method step S1. The clinical data structure 100 also defines transitions between said several clinical states 1 to 4, thus allowing for different paths of the clinical procedure. One exemplary path is show in Figure 1 with the dashed line. The shown path defines a development of the clinical procedure along depicted clinical states 1, 2 and 3, but not along clinical state 4 of the clinical procedure shown in Figure 1. Note, however, that another possible path may extend in both directions, i.e., back and forth, of a transition between two states. Thus, one other possible path in the illustrative example of Figure 1 is the path from clinical state 1 to state 2 to state 3, back to state 2 and then to state 4. Furthermore, the geometrical models used in this example of Figure 1 comprise a plurality of geometrical model parameters and boundary conditions for said geometrical model parameters, respectively. This was explained hereinbefore in the context of method step S2, i.e., the provision of at least one geometrical model for modelling procedural scenes in one or more of said clinical states of said clinical procedure. One scene is defined or individualized when each parameter is defined with one value. For example, in the illustrative example of Figure 1, a "procedural scene" or "scene" of clinical state 1 is defined when both parameters q₁ and q₂ are defined or determined. In Figure 1 one particular, individualized scene of clinical state 1 is shown, in which q₁ is set to 0,9 and q₂ is set to be 0,7. q₁ also fulfils the pre-defined boundary conditions that the minimum value for q₁ is q₁, _{Min}=0,1 and that the maximum value for q₁ is q_{1, Max}=14. Thus, with the sensor values q₁=0,9 and q₂=0,7 one procedural scene of clinical state 1 is defined. For example, the parameter q₁ is *"position of the surgeon in the coordinate system of the operating room"* and the parameter q₂ is *"position of the tracked hip-joint implant in the coordinate system of the operating room".* As is appreciate by the skilled reader, the same holds true for procedural scenes defined by the exemplarily chosen parameter values, i.e., the exemplarily chosen sensor values, shown in Figure 1 for clinical states 2, 3 and 4.

Providing such a clinical data structure 100 as shown in Figure 1 as well as one or more geometrical models for modelling procedural scenes allows carrying out the method as presented herein. Thus, based on the clinical data structure 100 and the provided one or more geometrical models, the following steps can be carried out to:
a) define an individual path of the clinical procedure by selecting at least one transition between a first and a second clinical state of the clinical data structure (step S3);
b) provide as input for the geometrical model, a set of values of the geometrical model parameters of the at least one geometrical model for said first, second and third clinical states (states 1 to 3) of the defined individual path thereby modelling a first, a second and a third individual procedural scene (step S4), and
c) generate the artificial intra-operative sensor data of said individual path from state 1 over state 2 to state 3 based on the modelled first, second and third individual procedural scenes (step S5). In an exemplary embodiment, said generation of the artificial intra-operative sensor data in step S5 is carried out by rendering image data of an intra operative image sensor and/or of an intra-operative video sensor.

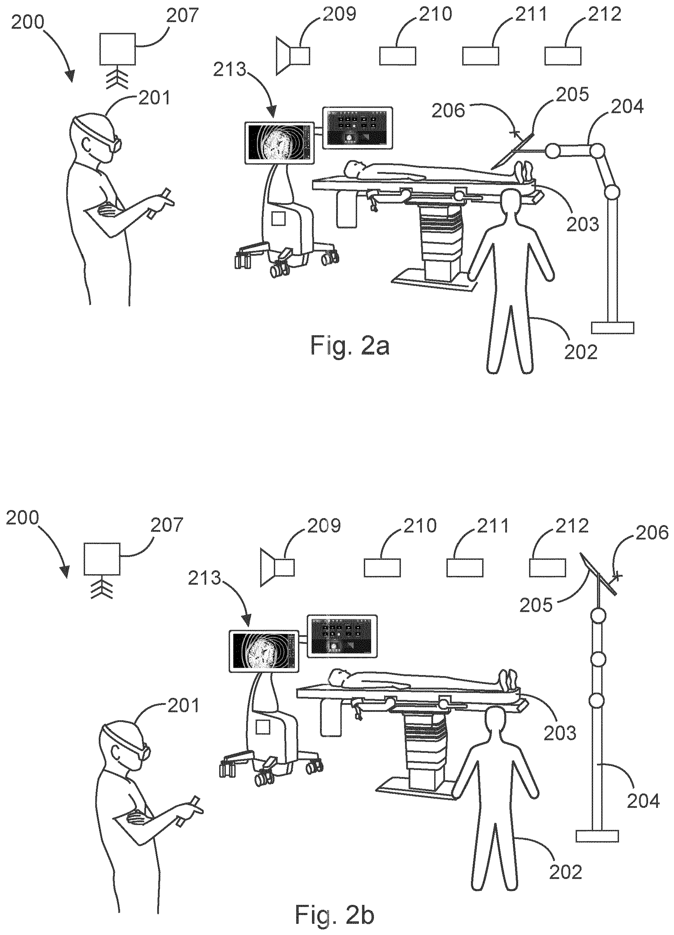

Figures 2a to 2c exemplarily show three different scenes of a surgical procedure in an operating room 200 in form of three different images, which were generated with a method embodiment of the present invention using at least steps S1 to S5 as described herein. Within the operating room 200 a first surgeon 201, a person of medical personal 202 as well as a patient 203 on a patient support is present. Furthermore, a robotic arm 204 holding the surgical instrument 205 is shown, which can be tracked by means of marker 206 and with the marker-based tracking system 207. Moreover, as further sensor the intra-operative image sensor 209 is shown as well as temperature sensor 210, sound sensor 211 as well as vibration sensor 212. The tracking system 207 can track the spatial position of the surgical instrument 205 by means electromagnetic radiotin 208 emitted and detected by the system 207 when reflected back. The system 207 can also track the position of the patient 203, of the surgeon 201 and of the medical personal 202 (respective markers not shown). The operating room 200 also comprises an intra-operative display 213 for illustrating medical images of the patient to the surgeon 201. The system 207 as well as sensors 209 to 212 are all intra operative sensors, the data of which can be modelled/simulated with the present invention. It is clear to the skilled reader that on the one hand these artificial sensors are used to generate the scenes of Figures 2a-2c, and on the other hand they are also shown in the rendered scenes. In other words, if a model that has been trained with the present invention is being used later to e.g. identify states, it uses the sensors 209 to 212 as input. As an example, we simulated tracking data of an instrument during a certain type of surgical step "A" performed by the surgeon, and when we later analyse tracking data during the real procedure from the sensor, we can identify that the surgical step "A" was performed. When comparing the three different scenes of the surgical procedure carried out in Figures 2a to 2c, one can see that the spatial position of the surgeon 201 in the operating room as well as the spatial position of the tracked surgical instrument 206 has changed from the scene shown in Figure 2a to Figure 2b. Further, in Figure 2c the surgeon 201 is not present anymore and the position of the surgical instrument 205 has again changed.

Figure 3 is a schematic illustration of different transitions of three sensor values transiting from a first to a second clinical state used in the context of the present invention. One could call the x-Axis "x" which is in line with the formula y(ax) mentioned above. Thus, in Figure 3 a geometrical model is used which is a function like e.g., y(ax), where the output y is the sensor value, which depends on variable input variable x. Illustrated is that two states are described by two geometrical models with potentially different parameters or boundary conditions; for at least some sensor values S (or called y in the formula), one can define a smooth transition between states, without having a dedicated geometrical model of the transition state. For example, one could have a first state "preparation" where one can simulate the entire OR as seen from a ceiling mounted camera, but without paying attention to anatomical detail. **In** a second state, one would simulate the surgical site, e.g., positions of organs and instruments; when switching between states, an instrument that is visible (e.g., position is simulated in) both states do not "jump" or at least stays substantially in place. The same holds true for the patient. Therefore, Figure 3 illustrates that a spatial registration of the second geometrical model into the first geometrical model can be used, where the spatial registration is configured to ensure that first sensor data of at least a first sensor generated for the first clinical state has a continuous transition into second sensor data of said at least first sensor generated for the second clinical state. This embodiment makes clear that the desired smooth transition holds true for all adjacent states, in which the respective models use or share at least one sensor of the same kind. In general, there should be smooth transitions of sensor data between adjacent or neighboured clinical states for sensors sensing or measuring spatial parameters as well as also for sensors sensing or measuring non-spatial parameters. In this way, one can achieve the result that for example a video rendered along the path of the clinical data structure describing the clinical procedure is consistent, e.g., the colour of one object is the same; the position of an object does not "jump", the type of object remains the same. However, as has been explained hereinbefore, also some parameters are allowed to jump to avoid training the model on the wrong parameters. Some parameters will also "jump" in reality, e.g. the intensity of light when the lamp is switched off. The presented method allows taking this into account.

Other variations to the disclosed embodiments can be understood and effected by those skilled in the art in practicing the claimed invention, from the study of the drawings, the disclosure, and the appended claims. In the claims the word "comprising" does not exclude other elements or steps and the indefinite article "a" or "an" does not exclude a plurality. A single processor or other unit may fulfil the functions of several items or steps recited in the claims. The mere fact that certain measures are recited in mutually different dependent claims does not indicate that a combination of these measures cannot be used to advantage. Any reference signs in the claims should not be construed as limiting the scope of the claims.

## Claims

1. A computer-implemented method of generating artificial intra-operative sensor data usable in training an artificial intelligence (AI) module, i.e., a machine learning model, the method comprising the following steps:
a) providing a clinical data structure describing several clinical states of a clinical procedure and defining transitions between said several clinical states allowing for different paths of the clinical procedure (step S1);
b) providing at least one geometrical model for modelling procedural scenes in one or more of said clinical states of said clinical procedure (step S2),
wherein the at least one geometrical model comprises a plurality of geometrical model parameters and boundary conditions for said geometrical model parameters, the boundary conditions being used to generate physically valid configurations only;
wherein the geometrical model parameters describe said procedural scenes in said clinical states of said clinical procedure,
wherein a particular combination of values of the geometrical model parameters defines a particular procedural scene of a clinical state of said clinical procedure; the method further comprising the steps
c) defining an individual path of the clinical procedure by selecting at least one transition between a first and a second clinical state of the clinical data structure (step S3);
d) providing, as input for the geometrical model, a set of values of the geometrical model parameters of the at least one geometrical model for said first and said second clinical states of the defined individual path thereby modelling a first individual procedural scene and a second individual procedural scene (step S4), and
e) generating the artificial intra-operative sensor data of said individual path based on the modelled first and second individual procedural scenes (step S5),
the method further comprising the steps
f) using the generated artificial intra-operative sensor data and the set of values of the geometric model parameters used during the generation of said artificial intra-operative sensor data for training an AI module for automatically analysing real intra-operative sensor data,
g) providing real intra-operative sensor data describing a real clinical procedure to the trained AI module trained with the generated artificial intra-operative sensor data,
and
h) automatically identifying said real clinical procedure by said trained AI module.

2. Method according to claim 1, the method further comprising the step
storing the generated artificial intra-operative sensor data of said individual path together with the set of values of the geometrical model parameters under which it was generated, wherein the set of values of the geometrical model parameters under which it was generated are stored as training labels; and/or
storing the generated artificial intra-operative sensor data of said individual path together with desired results an AI module trained on the data should produce, wherein the desired results an AI module trained on the data should produce are stored as training labels.

3. Method according to claim 1 or 2,
wherein each clinical state of the clinical data structure comprises a label, and
wherein each label describes the respective clinical state as a characteristic phase of said clinical procedure and describes it in a clinically differentiable manner.

4. Method according to any of the preceding claims,
wherein for the first clinical state of said individual path a first geometrical model is provided and wherein for the second clinical state of said individual path a second geometrical model is provided, and
wherein the first and second geometrical models are different from another, and/or
wherein at least the boundary conditions of the first and second model are different, wherein the second geometrical model is spatially registered into the first geometrical model,
wherein the spatial registration of the second geometrical model into the first geometrical model is configured to ensure that first sensor data of at least a first sensor generated for the first clinical state have a continuous transition into second sensor data of said at least first sensor generated for the second clinical state, and
wherein for all geometrical models of all adjacent clinical states of the clinical data structure, which are used to generate artificial intra-operative sensor data for the same type of artificial intra-operative sensor, said spatial registration of the respective geometrical model is configured to ensure said continuous transition of the generated sensor data of said adjacent clinical state.

5. Method according to any of the preceding claims,
wherein artificial intra-operative sensor data are generated for all clinical states of said defined individual path of the clinical procedure.

6. Method according to any of the preceding claims,
wherein the sensor data are intra-operative video data, and
wherein the step S5 of generating the artificial sensor data comprises rendering an artificial video of said clinical procedure.

7. Method according to any of the preceding claims,
wherein the clinical data structure is embodied as a graph, in which one path comprises a plurality of discrete states of the clinical procedure.

8. Method according to any of the preceding claims,
wherein each transition of the clinical data structure includes a weight associated with a frequency of occurrence in the clinical procedure.

9. Method according to any of the preceding claims,
wherein the clinical procedure is a surgical procedure, a diagnostic procedure, and/or a therapeutic procedure.

10. Method according to any of the preceding claims,
wherein for at least two clinical states, preferably for all clinical states, the respective geometrical model, the respective geometrical model parameters and the respective boundary conditions are dependent on the clinical state.

11. Method according to any of the preceding claims,
wherein the provided geometrical model is configured for spatially modelling procedural scenes in one or more of said clinical states of said clinical procedure.

12. Method according to any of the preceding claims,
wherein the geometrical model parameters define at least a position, a shape and/or a type of one or more objects occurring in said procedural scene of said clinical state of said clinical procedure,

13. A program which, when running on a computer or when loaded onto a computer, causes the computer to perform the method steps of the method according to any of the preceding claims.

14. A program storage medium on which the program of claim 13 and/or training data for training an AI module for automatically analysing real intra-operative sensor data is stored, wherein the training data were generated according to the method of any of the claims 1 to 12.

15. An AI module, i.e., a machine learning model, for automatically analysing real intra-operative sensor data,
wherein the AI module was trained with training data generated according to the method of any of the claims 1 to 12; wherein the AI module is configured to receive as an input real intra-operative sensor data describing a real clinical procedure, and
wherein the AI module is configured for automatically identifying said real clinical procedure by analysing the received real intra-operative sensor data.

## Patentansprüche

1. Computerimplementiertes Verfahren zur Erzeugung künstlicher intraoperativer Sensordaten, die zum Training eines Moduls für künstliche Intelligenz (KI), d.h. eines maschinellen Lernmodells, verwendbar sind, wobei das Verfahren die folgenden Schritte aufweist:
a) Bereitstellen einer klinischen Datenstruktur, die mehrere klinische Zustände eines klinischen Verfahrens beschreibt und Übergänge zwischen den mehreren klinischen Zuständen definiert, die verschiedene Pfade des klinischen Verfahrens ermöglichen (Schritt S1);
b) Bereitstellen mindestens eines geometrischen Modells zur Modellierung von Verfahrensszenen in einem oder mehreren der klinischen Zustände des klinischen Verfahrens (Schritt S2),
wobei das mindestens eine geometrische Modell eine Vielzahl von geometrischen Modellparametern und Randbedingungen für die geometrischen Modellparameter aufweist, wobei die Randbedingungen verwendet werden, um ausschließlich physikalisch gültige Konfigurationen zu erzeugen;
wobei die geometrischen Modellparameter die Verfahrensszenen in den klinischen Zuständen des klinischen Verfahrens beschreiben,
wobei eine bestimmte Kombination von Werten der geometrischen Modellparameter eine bestimmte Verfahrensszene eines klinischen Zustands des klinischen Verfahrens definiert; wobei das Verfahren ferner die folgenden Schritte aufweist
c) Definieren eines individuellen Pfades des klinischen Verfahrens durch Auswählen mindestens eines Übergangs zwischen einem ersten und einem zweiten klinischen Zustand der klinischen Datenstruktur (Schritt S3);
d) Bereitstellen, als Eingabe für das geometrische Modell, eines Satzes von Werten der geometrischen Modellparameter des mindestens einen geometrischen Modells für den ersten und den zweiten klinischen Zustand des definierten individuellen Pfades, wodurch eine erste individuelle Verfahrensszene und eine zweite individuelle Verfahrensszene modelliert werden (Schritt S4), und
e) Erzeugen der künstlichen intraoperativen Sensordaten des individuellen Pfades basierend auf den modellierten ersten und zweiten individuellen Verfahrensszenen (Schritt S5),
wobei das Verfahren ferner die folgenden Schritte aufweist
f) Verwenden der erzeugten künstlichen intraoperativen Sensordaten und des Satzes von Werten der geometrischen Modellparameter, die während der Erzeugung der künstlichen intraoperativen Sensordaten verwendet wurden, zum Training eines KI-Moduls zur automatischen Analyse realer intraoperativer Sensordaten,
g) Bereitstellen realer intraoperativer Sensordaten, die ein reales klinisches Verfahren beschreiben, an das trainierte KI-Modul, das mit den erzeugten künstlichen intraoperativen Sensordaten trainiert wurde, und
h) automatisches Identifizieren des realen klinischen Verfahrens durch das trainierte Kl-Modul.

2. Verfahren nach Anspruch 1, wobei das Verfahren ferner den Schritt aufweist Speichern der erzeugten künstlichen intraoperativen Sensordaten des individuellen Pfades zusammen mit dem Satz von Werten der geometrischen Modellparameter, unter denen sie erzeugt wurden, wobei der Satz von Werten der geometrischen Modellparameter, unter denen sie erzeugt wurden, als Trainingslabels gespeichert wird; und/oder
Speichern der erzeugten künstlichen intraoperativen Sensordaten des individuellen Pfades zusammen mit gewünschten Ergebnissen, die ein mit den Daten trainiertes KI-Modul erzeugen soll, wobei die gewünschten Ergebnisse, die ein mit den Daten trainiertes KI-Modul erzeugen soll, als Trainingslabels gespeichert werden.

3. Verfahren nach Anspruch 1 oder 2,
wobei jeder klinische Zustand der klinischen Datenstruktur ein Label aufweist, und wobei jedes Label den jeweiligen klinischen Zustand als charakteristische Phase des klinischen Verfahrens beschreibt und ihn in klinisch unterscheidbarer Weise beschreibt.

4. Verfahren nach einem der vorhergehenden Ansprüche,
wobei für den ersten klinischen Zustand des individuellen Pfades ein erstes geometrisches Modell bereitgestellt wird und wobei für den zweiten klinischen Zustand des individuellen Pfades ein zweites geometrisches Modell bereitgestellt wird, und wobei das erste und das zweite geometrische Modell voneinander verschieden sind, und/oder wobei zumindest die Randbedingungen des ersten und des zweiten Modells verschieden sind, wobei das zweite geometrische Modell räumlich in das erste geometrische Modell registriert ist,
wobei die räumliche Registrierung des zweiten geometrischen Modells in das erste geometrische Modell so konfiguriert ist, dass erste Sensordaten mindestens eines ersten Sensors, die für den ersten klinischen Zustand erzeugt wurden, einen kontinuierlichen Übergang in zweite Sensordaten des mindestens ersten Sensors aufweisen, die für den zweiten klinischen Zustand erzeugt wurden, und
wobei für alle geometrischen Modelle aller benachbarten klinischen Zustände der klinischen Datenstruktur, die verwendet werden, um künstliche intraoperative Sensordaten für denselben Typ von künstlichem intraoperativem Sensor zu erzeugen, die räumliche Registrierung des jeweiligen geometrischen Modells so konfiguriert ist, dass der kontinuierliche Übergang der erzeugten Sensordaten des benachbarten klinischen Zustands sichergestellt wird.

5. Verfahren nach einem der vorhergehenden Ansprüche,
wobei künstliche intraoperative Sensordaten für alle klinischen Zustände des definierten individuellen Pfades des klinischen Verfahrens erzeugt werden.

6. Verfahren nach einem der vorhergehenden Ansprüche,
wobei die Sensordaten intraoperative Videodaten sind, und
wobei der Schritt S5 des Erzeugens der künstlichen Sensordaten das Rendern eines künstlichen Videos des klinischen Verfahrens aufweist.

7. Verfahren nach einem der vorhergehenden Ansprüche,
wobei die klinische Datenstruktur als Graph ausgeführt ist, in dem ein Pfad eine Vielzahl von diskreten Zuständen des klinischen Verfahrens aufweist.

8. Verfahren nach einem der vorhergehenden Ansprüche,
wobei jeder Übergang der klinischen Datenstruktur ein Gewicht enthält, das mit einer Häufigkeit des Auftretens im klinischen Verfahren assoziiert ist.

9. Verfahren nach einem der vorhergehenden Ansprüche,
wobei das klinische Verfahren ein chirurgisches Verfahren, ein diagnostisches Verfahren und/oder ein therapeutisches Verfahren ist.

10. Verfahren nach einem der vorhergehenden Ansprüche,
wobei für mindestens zwei klinische Zustände, vorzugsweise für alle klinischen Zustände, das jeweilige geometrische Modell, die jeweiligen geometrischen Modellparameter und die jeweiligen Randbedingungen vom klinischen Zustand abhängig sind.

11. Verfahren nach einem der vorhergehenden Ansprüche,
wobei das bereitgestellte geometrische Modell zur räumlichen Modellierung von Verfahrensszenen in einem oder mehreren der klinischen Zustände des klinischen Verfahrens konfiguriert ist.

12. Verfahren nach einem der vorhergehenden Ansprüche,
wobei die geometrischen Modellparameter mindestens eine Position, eine Form und/oder einen Typ eines oder mehrerer Objekte definieren, die in der Verfahrensszene des klinischen Zustands des klinischen Verfahrens auftreten.

13. Programm, das, wenn es auf einem Computer läuft oder auf einen Computer geladen wird, den Computer veranlasst, die Verfahrensschritte des Verfahrens nach einem der vorhergehenden Ansprüche auszuführen.

14. Programmspeichermedium, auf dem das Programm nach Anspruch 13 und/oder Trainingsdaten zum Training eines KI-Moduls zur automatischen Analyse realer intraoperativer Sensordaten gespeichert sind, wobei die Trainingsdaten gemäß dem Verfahren nach einem der Ansprüche 1 bis 12 erzeugt wurden.

15. KI-Modul, d.h. ein maschinelles Lernmodell, zur automatischen Analyse realer intraoperativer Sensordaten,
wobei das KI-Modul mit Trainingsdaten trainiert wurde, die gemäß dem Verfahren nach einem der Ansprüche 1 bis 12 erzeugt wurden; wobei das KI-Modul konfiguriert ist, als Eingabe reale intraoperative Sensordaten zu empfangen, die ein reales klinisches Verfahren beschreiben, und
wobei das KI-Modul zur automatischen Identifizierung des realen klinischen Verfahrens durch Analyse der empfangenen realen intraoperativen Sensordaten konfiguriert ist.

## Revendications

1. Procédé mis en œuvre par ordinateur pour générer des données de capteur peropératoire artificiel utilisables dans l'apprentissage d'un module d'intelligence artificielle (AI), c'est-à-dire d'un modèle d'apprentissage automatique, le procédé comprenant les étapes suivantes :
a) fournir une structure de données cliniques décrivant plusieurs états cliniques d'une procédure clinique et définissant des transitions entre lesdits plusieurs états cliniques permettant différents parcours de la procédure clinique (étape S1) ;
b) fournir au moins un modèle géométrique pour modéliser des scènes procédurales dans un ou plusieurs desdits états cliniques de ladite procédure clinique (étape S2),
dans lequel ledit au moins un modèle géométrique comprend une pluralité de paramètres de modèle géométrique et des conditions de limite pour lesdits paramètres de modèle géométrique, les conditions de limite étant utilisées pour générer des configurations physiquement valides uniquement ;
dans lequel les paramètres de modèle géométrique décrivent lesdites scènes procédurales dans lesdits états cliniques de ladite procédure clinique,
dans lequel une combinaison particulière de valeurs des paramètres de modèle géométrique définit une scène procédurale particulière d'un état clinique de ladite procédure clinique ; le procédé comprenant en outre les étapes de :
c) définir un parcours individuel de la procédure clinique en sélectionnant au moins une transition entre un premier et un second état clinique de la structure de données cliniques (étape S3) ;
d) fournir, en tant qu'élément d'entrée pour le modèle géométrique, un ensemble de valeurs des paramètres de modèle géométrique dudit au moins un modèle géométrique pour ledit premier et ledit second états cliniques du parcours individuel défini, en modélisant ainsi une première scène procédurale individuelle et une seconde scène procédurale individuelle (étape S4), et
e) générer les données de capteur peropératoire artificiel dudit parcours individuel sur la base des première et seconde scènes procédurales individuelles modélisées (étape S5),
le procédé comprenant en outre les étapes de :
f) utiliser les données de capteur peropératoire artificiel générées et l'ensemble de valeurs des paramètres de modèle géométrique utilisés pendant la génération desdites données de capteur peropératoire artificiel pour apprendre à un module AI à analyser automatiquement des données de capteur peropératoire réelles,
g) fournir des données de capteur peropératoire réelles décrivant une procédure clinique réelledit au module AI entraîné, entraîné avec les données de capteur peropératoire artificiel générées,
et
h) identifier automatiquement ladite procédure clinique réelle par ledit module AI entraîné.

2. Procédé selon la revendication 1, le procédé comprenant en outre l'étape de :
stocker les données de capteur peropératoire artificiel générées dudit parcours individuel en association avec l'ensemble de valeurs des paramètres de modèle géométrique sous lesquelles elles ont été générées, dans lequel l'ensemble de valeurs des paramètres de modèle géométrique sous lesquelles elles ont été générées est stocké sous forme d'étiquettes d'apprentissage ; et/ou
stocker les données de capteur peropératoire artificiel générées dudit parcours individuel en association avec des résultats voulus qu'un module AI entraîné sur les données doit produire, dans lequel les résultats voulus qu'un module AI entraîné sur les données doit produire sont stockés sous forme d'étiquettes d'apprentissage.

3. Procédé selon la revendication 1 ou 2,
dans lequel chaque état clinique de la structure de données cliniques comprend une étiquette, et
dans lequel chaque étiquette décrit l'état clinique respectif comme une phase caractéristique de ladite procédure clinique et le décrit d'une manière différenciable du point de vue clinique.

4. Procédé selon l'une quelconque des revendications précédentes,
dans lequel un premier modèle géométrique est fourni pour le premier état clinique dudit parcours individuel et dans lequel un second modèle géométrique est fourni pour le second état clinique dudit parcours individuel, et
dans lequel les premier et second modèles géométriques sont différents l'un de l'autre et/ou
dans lequel au moins les conditions de limite des premier et second modèles sont différentes,
dans lequel le second modèle géométrique est enregistré spatialement dans le premier modèle géométrique,
dans lequel l'enregistrement spatial du second modèle géométrique dans le premier modèle géométrique est configuré pour garantir que des premières données de capteur d'au moins un premier capteur générées pour le premier état clinique présentent une transition continue dans des secondes données de capteur dudit au moins premier capteur générées pour le second état clinique, et
dans lequel, pour tous les modèles géométriques de tous les états cliniques adjacents de la structure de données cliniques, qui sont utilisés pour générer des données de capteur peropératoire artificiel pour le même type de capteur peropératoire artificiel, ledit enregistrement spatial du modèle géométrique respectif est configuré pour garantir ladite transition continue des données de capteur générées dudit état clinique adjacent.

5. Procédé selon l'une quelconque des revendications précédentes,
dans lequel des données de capteur artificiel peropératoire sont générées pour tous les états cliniques dudit parcours individuel défini de la procédure clinique.

6. Procédé selon l'une quelconque des revendications précédentes,
dans lequel les données de capteur sont des données vidéo peropératoires, et
dans lequel l'étape S5 de génération des données de capteur artificiel comprend d'effectuer un rendu d'une vidéo artificielle de ladite procédure clinique.

7. Procédé selon l'une quelconque des revendications précédentes,
dans lequel la structure de données cliniques est incorporée sous la forme d'un graphique, dans lequel un parcours comprend une pluralité d'états discrets de la procédure clinique.

8. Procédé selon l'une quelconque des revendications précédentes,
dans lequel chaque transition de la structure de données cliniques inclut une pondération associée à une fréquence d'occurrence dans la procédure clinique.

9. Procédé selon l'une quelconque des revendications précédentes,
dans lequel la procédure clinique est une procédure chirurgicale, une procédure de diagnostic, et/ou une procédure thérapeutique.

10. Procédé selon l'une quelconque des revendications précédentes,
dans lequel, pour au moins deux états cliniques, de préférence pour tous les états cliniques, le modèle géométrique respectif, les paramètres de modèle géométrique respectifs, et les conditions de limite respectives dépendent de l'état clinique.

11. Procédé selon l'une quelconque des revendications précédentes,
dans lequel le modèle géométrique fourni est configuré pour modéliser spatialement des scènes procédurales dans un ou plusieurs desdits états cliniques de ladite procédure clinique.

12. Procédé selon l'une quelconque des revendications précédentes,
dans lequel les paramètres de modèle géométrique définissent au moins une position, une forme, et/ou un type d'un ou plusieurs objets apparaissant dans ladite scène procédurale dudit état clinique de ladite procédure clinique.

13. Programme qui, lorsqu'il s'exécute sur un ordinateur ou lorsqu'il est chargé sur un ordinateur, amène l'ordinateur à réaliser les étapes de procédé du procédé selon l'une quelconque des revendications précédentes.

14. Support de stockage de programme sur lequel est stocké le programme de la revendication 13 et/ou des données d'apprentissage destinées à entraîner un module AI à analyser automatiquement des données réelles de capteur peropératoire, dans lequel les données d'apprentissage ont été générées selon le procédé de l'une quelconque des revendications 1 à 12.

15. Module AI, c'est-à-dire un modèle d'apprentissage automatique, destiné à analyser automatiquement des données réelles de capteur peropératoire,
dans lequel le module AI a été entraîné avec des données d'apprentissage générées selon le procédé de l'une quelconque des revendications 1 à 12 ; dans lequel le module AI est configuré pour recevoir, en tant qu'élément d'entrée, des données réelles de capteur peropératoire décrivant une procédure clinique réelle, et
dans lequel le module AI est configuré pour identifier automatiquement ladite procédure clinique réelle en analysant les données réelles de capteur peropératoire reçues.
